# EUROPEAN PATENT SPECIFICATION

(11) **EP 1 961 772 B1**
(45) Date of publication and mention of the grant of the patent: **10.04.2013**
(21) Application number: 06832873.1
(22) Date of filing: 17.11.2006
(51) Int. Cl.: C08B 37/08, A61L 27/00

(54) **CATIONIZED HYALURONIC ACID**
KATIONISIERTE HYALURONSÄURE
ACIDE HYALURONIQUE CATIONISE

(30) Priority: 01.12.2005 JP 2005347501
(43) Date of publication of application: 27.08.2008
(73) Proprietor: Shiseido Company, Limited, Tokyo 104-8010 (JP)
(72) Inventor: MORI, Yuichiro, Yokohama-shi, Kanagawa 2248558 (JP); MATSUMOTO, Tetsunori, Yokohama-shi, Kanagawa 2368643 (JP); YOKOKAWA, Yoshihiro, Yokohama-shi, Kanagawa 2248558 (JP)
(74) Representative: Henkel, Breuer & Partner
(86) International application number: PCT/JP2006/322995
(87) International publication number: WO 2007/063725

(56) References cited:
- JP-A- 07 033 682
- JP-A- 09 012 589
- JP-A- 2002 000 638
- JP-A- 2003 064 102
- JP-A- 2006 117 746

## Description

### FIELD OF THE INVENTION

The present invention relates to a cationized hyaluronic acid wherein a cationic group is introduced into hyaluronic acid.

### BACKGROUND OF THE INVENTION

Hyaluronic acid is a polymer wherein an N-acetyl-D-glucosamine and a D-glucuronic acid are bonded together to form one unit, and a number of units are repeatedly bonded as represented by the following formula (I).

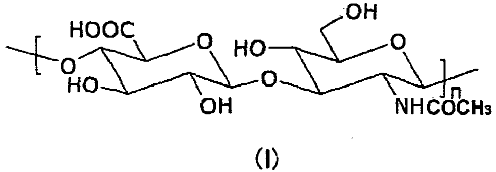

Hyaluronic acid has a polyanionic property owning to the presence of carboxyl groups derived from glucuronic acid. It is known that, using such a polyanionic property, a water- insoluble polyion complex gel can be formed by combining hyaluronic acid with a polycationic substance in water.
In Patent Literature 1, for example, a hyaluronic acid gel capsule enclosing a drug, in which a polyion complex film is formed with a cationic substance on the surface of hyaluronic acid gel, is disclosed.
Additionally, in Patent Literature 2, a sustained-release preparation using a polyion complex of hyaluronic acid and a cationic polyacrylic acid derivative as a pharmaceutical carrier.
Furthermore, applications for medical materials such as medical adhesives, fixing agents and adhesion preventing agents have been reported in Patent Literatures 3 to 5.

Hyaluronic acid is excellent in biological safety and biocompatibility because it is widely distributed in connective tissues of a living body, including a human. It is desired that a cationic substance partnered with hyaluronic acid in forming a polyion complex is also excellent in biological safety and biocompatibility.
Chitosan, which is a deacetylated chitin, and aminated cellulose are known as cationic polymers derived from living body material. However, a cationic polymer much closer to the component of a human body has been desired.
Patent Literature 1 : Japanese Unexamined Patent Publication H06-254381
Patent Literature 2 : Japanese Unexamined Patent Publication H07-33682
Patent Literature 3 :Japanese Unexamined Patent Publication 2000-5296
Patent Literature 4 :Japanese Unexamined Patent Publication 2000-116765
Patent Literature 5 :Japanese Unexamined Patent Publication 2002-638

### DISCLOSURE OF THE INVENTION

### PROBLEM TO BE SOLVED BY THE INVENTION

The present invention was done in view of the aforementioned problem of the prior art, and an object thereof is to provide a cationic polymer which is much closer to the component of a human body, and capable of forming a polyion complex together with an anionic biocompatible material such as hyaluronic acid.

### MEANS TO SOLVE THE PROBLEM

In order to attain the aforementioned object, the present inventors tried introducing a quaternary ammonium cation group into hyaluronic acid and succeeded in obtaining a cationized hyaluronic acid. Then, the present inventors found that the cationized hyaluronic acid can form a polyion complex gel together with hyaluronic acid in the presence of water, which resulted in completion of the present invention.
That is, a cationized hyaluronic acid according to the present invention is a hyaluronic acid wherein at least one part of hydroxylic hydrogen atoms of hyaluronic acid is replaced with a group having a quaternary ammonium cation group.
The cationized hyaluronic acid of the present invention is preferably represented by the following formula (1):

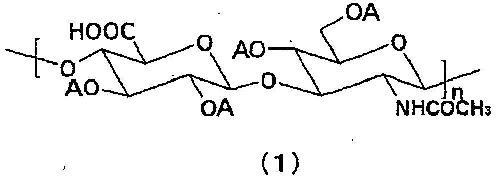

wherein A represents a hydrogen atom or a substituent represented by the following formula (2):

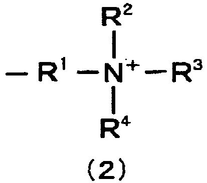

wherein R¹ represents an alkylene group having 3 to 5 carbon atoms which may have a hydroxyl group, and each R², R³ and R⁴ represents an alkyl group having 1 to 3 carbon atoms; and
an average degree of substitution with the substituent is 0.1 or more per one unit of hyaluronic acid.

Additionally, the cationized hyaluronic acid of the present invention is preferably obtained by reacting hyaluronic acid with a cationizing agent represented by the following formula (3): 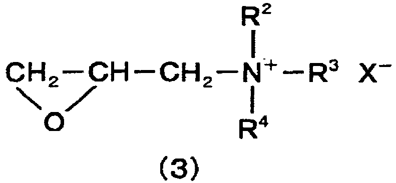 wherein each R², R³ and R⁴ represents an alkyl group having 1 to 3 carbon atoms, and X represents a halogen atom.

The polyion complex according to the present invention is formed with any of the aforementioned cationized hyaluronic acid and an anionic biocompatible material. Preferable anionic biocompatible materials include hyaluronic acid.

The cationized hyaluronic acid of the present invention can form a polyion complex with an anionic material such as hyaluronic acid in the presence of water and can be expected to be applied for various kinds of applications where a hydrogel is conventionally used. For example, the polyion complex of the cationized hyaluronic acid with hyaluronic acid is a water- insoluble hydrogel and mainly consists of hyaluronic acid which exists in a human body. Thus, it has excellent biological safety and biocompatibility and is preferable in the fields such as pharmaceutical, medical care, sanitation, food and cosmetics.

### BRIEF DESCRIPTION OF THE DRAWINGS

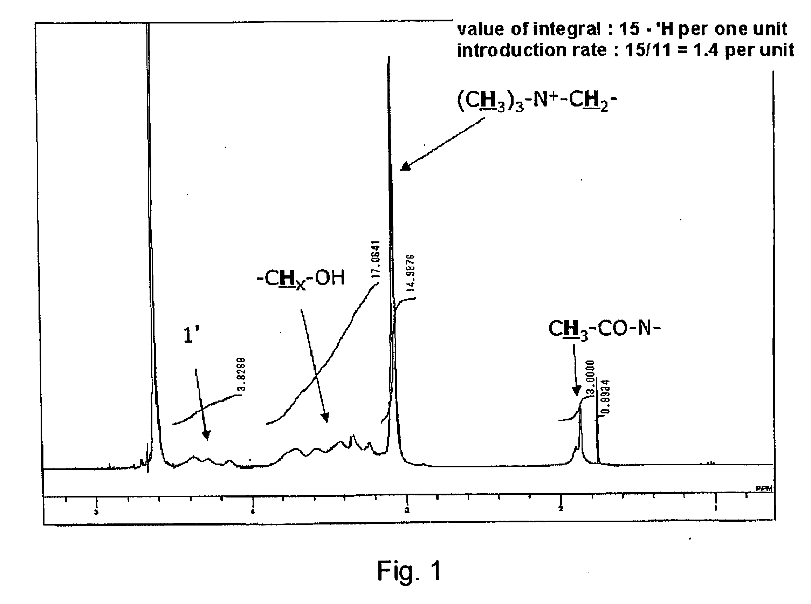

Figure 1 is a ¹H-NMR spectrum of a cationized hyaluronic acid (Preparation Example 1) according to the present invention.

### BEST MODE FOR CARRYING OUT THE INVENTION

A cationized hyaluronic acid of the present invention is a hyaluronic acid wherein at least one part of hydroxylic hydrogen atoms of hyaluronic acid is replaced with a group having a quaternary ammonium cation group.
Preferable examples for the cationized hyaluronic acid of the present invention include a cationized hyaluronic acid having the structure represented by the aforementioned formula (1).

In formula (1), A represents a hydrogen atom or a substituent represented by the aforementioned formula (2). An average degree of substitution with the substituent represented by formula (2) is preferably 0.1 or more per one unit of hyaluronic acid, and more preferably 1 or more per one unit.
In formula (2), R¹ represents an alkylene group having 3 to 5 carbon atoms and may have a hydroxy group. Preferable examples for R¹ include -CH₂CH(OH)CH₂-.
Each R², R³ and R⁴ represents an alkyl group having 1 to 3 carbon atoms.
"n" is a positive integer representing a degree of polymerization. Though molecular weight of hyaluronic acid used is not particularly limited, it is normally from about 100,000 to about 3,000,000.

The cationized hyaluronic acid of the present invention can be synthesized by reacting hyaluronic acid with a cationizing agent.
A method for producing hyaluronic acid used as a starting material is not particularly limited. Hyaluronic acid is industrially manufactured by extraction from living tissue such as cockscomb or by microorganism cultivation. In addition, a hyaluronate, such as sodium salt, is commercially available. In the present invention, hyaluronic acid or salt thereof can be used.

The cationizing agent is not particularly limited as long as it is a compound having a quaternary ammonium cation group and a reactive group to a hydroxyl group of hyaluronic acid. The amount of the cationizing agent used can be appropriately set depending on a desired degree of substitution: it is normally 0.1 times or more (molar ratio) per one unit of hyaluronic acid, and more preferably 1 times or more (molar ratio).

Preferred examples of cationizing agents include glycidyltrialkyleammonium halide represented in the aforementioned formula (3).
In formula (3), each R², R³ and R⁴ represents an alkyl group having 1 to 3 carbon atoms. X represents a halogen atom, including Cl, Br, I, and the like, preferably Cl. Specific examples include glycidyltrimethylammonium chloride, glycidyltriethylammonium chloride and glycidyltripropylammonium chloride.

When glycidyltrialkyleammonium halide is used as a cationizing agent, the reaction is preferably carried out in the presence of alkali. Examples for alkali include alkali metal hydroxides such as sodium hydroxide and potassium hydroxide; alkaline earth metal hydroxides such as calcium hydroxide and magnesium hydroxide; and organic amines such as ethylenediamine, triethylamine and trimethylamine: preferably sodium hydroxide.

Generally, water is preferably used as a solvent. Other solvents, however, may be used as long as the reaction is not particularly interrupted.
The reaction temperature is appropriately set depending on the starting materials or a desired degree of substitution: it is normally from 20 to 50 °C, preferably from 25 to 40 °C. Because hydrolysis of hyaluronic acid may progress to reduce molecular weight thereof at high temperature, a careful attention is required when the reduction of molecular weight is not desired.

After completion of the reaction, a cationized hyaluronic acid is precipitated by adding a poor solvent for the cationized hyaluronic acid, such as alcohol or acetone, to the reaction mixture. If necessary, the precipitate is washed and dried to obtain the cationized hyaluronic acid of the present invention.

A cationized hyaluronic acid has a polycationic property, while usual hyaluronic acid has a polyanionic property. Thus, when a cationized hyaluronic acid and hyaluronic acid coexist in water, a polyion complex is formed and precipitated as a water-insoluble gel by electrostatic interaction between them. The gel can be formed into various kinds of shapes such as granule, thin-film and block, depending on purpose. The gel may be used in a hydrogel state as it is or in dried state.
The mixture ratio of a cationized hyaluronic acid and hyaluronic acid is not particularly limited as long as it is within the range of forming a polyion complex, and can be appropriately decided depending on purpose.

In addition, a drug can be enclosed inside the gel when a cationized hyaluronic acid and hyaluronic acid coexist in water, in which the drug is dissolved or dispersed. Alternatively, a drug can be enclosed inside the gel by a method that a hyaluronic acid aqueous solution containing a water-soluble drug dissolved therein is added dropwise into a cationized hyaluronic acid aqueous solution, or a method that an emulsion, in which oil phase comprising an oil-soluble drug is emulsified and dispersed in a hyaluronic acid aqueous solution, is added dropwise into a cationized hyaluronic acid aqueous solution, according to methods described in Patent Literature 1.

Because the polyion complex of the present invention mainly consists of hyaluronic acid, it has excellent biological safety and biocompatibility and can be utilized such as pharmaceutical carrier. Additionally, it is also possible to utilize the polyion complex of the present invention as materials for medical, sanitary, food and cosmetics.
Depending on purpose or application, it is also possible to use the cationized hyaluronic acid of the present invention in forming a polyion complex with other anionic substances. Other anionic substances are not limited as long as they have a number of intramolecular anionic groups, such as carboxyl group and sulfate group, and form a polyion complex with the cationized hyaluronic acid in the presence of water.

Examples of anionic substances include anionic natural polysaccharides such as alginic acid, chondroitin sulfate, dextran sulfate and pectine; anionic synthetic polysaccharides such as carboxymethylcellulose, carboxymethyldextran, carboxymethyl starch, carboxymethylchitosan, sulfated cellulose and sulfated dextran; acidic amino acid polymers such as polyglutamic acid, polyaspartic acid and glutamic-aspartic acid copolymer; polynucleotides; acidic proteins such as serum albumin, pepsine, urease and fetuin; anionic polymers such as polyacrylic acid and carboxy vinyl polymer; and derivatives and salts thereof. Additionally, anionic biocompatible polymers having a phosphorylcholine group are also included.
The present invention will be explained in detail below by way of embodiments, but not to be limited thereto.

### EXAMPLES

### Preparation Example 1: Production of cationized hyaluronic acid

10.4 mL (0.078mol) of glycidyltrimethylammonium chloride was mixed with 11.25 mL of water, and then, 5g (0.013mol as a disaccharide unit) of hyaluronic acid (BIO HYARO 9^{γM}: manufactured by Shiseido Company, Ltd.) and 1.25 mL of 2M aqueous sodium hydroxide solution were added and dissolved thereto.
The mixture solution was reacted for three days at room temperature. After completion of the reaction, a reaction product was precipitated with methanol added thereto, and then sufficiently washed with acetone, to give 4.2g of white powder.
The obtained powder was subjected to GPC, to comfirm that low-molecule compounds (unreacted reagents) have been removed.
Additionally, in ¹H-NMR (See Fig. 1), a signal derived from proton of trimethylammonium was detected, whereby it was confirmed that 2-hydroxy-3-trimethylammoniumpropyl group was introduced thereinto.
The average degree of substitution calculated from the value of integral in ¹H-NMR was about 1.4 per one unit of hyaluronic acid.

### Test Example 1 : Formation of polyion complex

1% (w/w) aqueous solution of the cationized hyaluronic acid obtained in Preparation Example 1 was prepared. 1% (w/w) aqueous solution of hyaluronic acid (BIO HYARO 12^{™}: manufactured by Shiseido Company, Ltd.) was separately prepared.
Though both solutions were transparent liquid in separated state, equal amount of them were combined to result in precipitating a gel. This gel is considered as a polyion complex in which cation groups in the cationized hyaluronic acid and anion groups in hyaluronic acid form ion pairs.

### Test Example 2 : Formation of polyion complex

1% (w/w) aqueous solution of the cationized hyaluronic acid obtained in Preparation Example 1 was prepared. 0.1% (w/w) aqueous solution of carboxy vinyl polymer was separately prepared.
Though both solutions were transparent liquid in separated state, equal amount of them were combined to result in precipitating a gel. This gel is considered as a polyion complex which cation groups in the cationized hyaluronic acid and anion groups in carboxy vinyl polymer form ion pairs.

## Claims

1. A cationized hyaluronic acid, wherein at least one part of hydroxylic hydrogen atoms of hyaluronic acid is replaced with a group having a quaternary ammonium cation group.

2. The cationized hyaluronic acid according to claim 1, wherein the cationized hyaluronic acid is represented by the following formula (1): 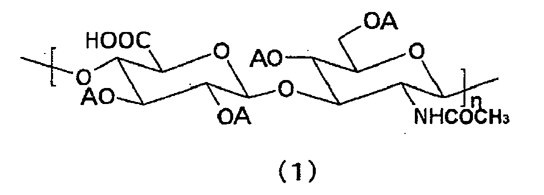 wherein A represents a hydrogen atom or a substituent represented by the following formula (2): 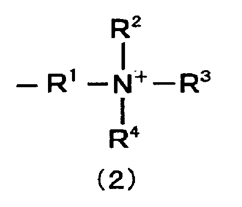 wherein R¹ represents an alkylene group having 3 to 5 carbon atoms which may have a hydroxyl group, and each R², R³ and R⁴ represents an alkyl group having 1 to 3 carbon atoms; and
an average degree of substitution with the substituent is 0.1 or more per one unit of hyaluronic acid.

3. The cationized hyaluronic acid according to claim 1 or 2, wherein the cationized hyaluronic acid is obtained by reacting hyaluronic acid with a cationizing agent represented by the following formula (3): 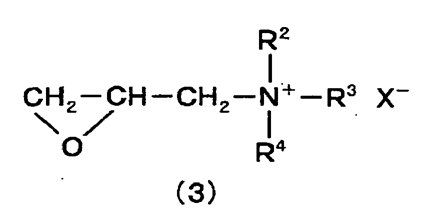 wherein each R², R³ and R⁴ represents an alkyl group having 1 to 3 carbon atoms, and X represents a halogen atom.

4. A polyion complex, which is formed with the cationized hyaluronic acid according to any of claims 1 to 3 and an anionic biocompatible material.

5. The polyion complex according to claim 4, wherein the anionic biocompatible material is hyaluronic acid.

## Patentansprüche

1. Kationisierte Hyaluronsäure, wobei mindestens ein Teil der Hydroxyl-Wasserstoffatome der Hyaluronsäure durch eine Gruppe mit einer quaternären Ammoniumkationengruppe ersetzt ist.

2. Kationisierte Hyaluronsäure nach Anspruch 1, wobei die kationisierte Hyaluronsäure durch die folgende Formel (1) dargestellt wird: 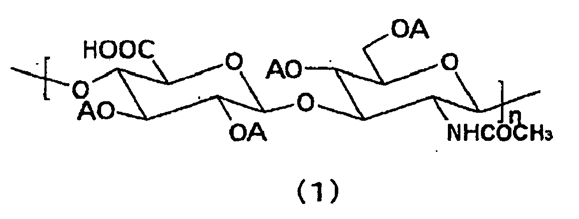 wobei A für ein Wasserstoffatom oder einen Substituenten der folgenden Formel (2) steht: 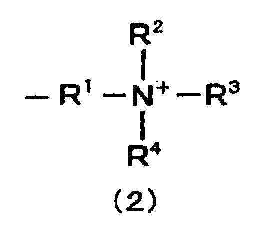 wobei R¹ für eine Alkylengruppe mit 3 bis 5 Kohlenstoffatomen, die eine Hydroxylgruppe aufweisen kann, steht und R², R³ und R⁴ jeweils für eine Alkylgruppe mit 1 bis 3 Kohlenstoffatomen stehen; und
der mittlere Substitutionsgrad mit dem Substituenten 0,1 oder mehr pro eine Einheit Hyaluronsäure beträgt.

3. Kationisierte Hyaluronsäure nach Anspruch 1 oder 2, wobei die kationisierte Hyaluronsäure dadurch erhalten wird, dass Hyaluronsäure mit einem Kationisierungsmittel der folgenden Formel (3) umgesetzt wird: 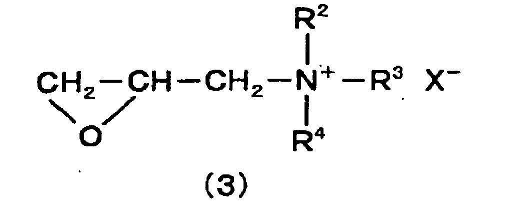 wobei R², R³ und R⁴ jeweils für eine Alkylgruppe mit 1 bis 3 Kohlenstoffatomen stehen und X für ein Halogenatom steht.

4. Polyionenkomplex, der mit der kationisierten Hyaluronsäure nach einem der Ansprüche 1 bis 3 und einem anionischen, biologisch kompatiblen Material gebildet ist.

5. Polyionenkomplex nach Anspruch 4, wobei das anionische, biologisch kompatible Material Hyaluronsäure ist.

## Revendications

1. Acide hyaluronique cationisé, dans lequel au moins une partie des atomes d'hydrogène hydroxyliques de l'acide hyaluronique est remplacée par un groupe ayant un groupe à cation ammonium quaternaire.

2. Acide hyaluronique cationisé selon la revendication 1, dans lequel l'acide hyaluronique cationisé est représenté par la formule (1) suivante : 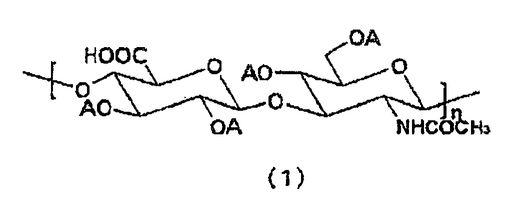 dans laquelle A représente un atome d'hydrogène ou un substituant représenté par la formule (2) suivante : 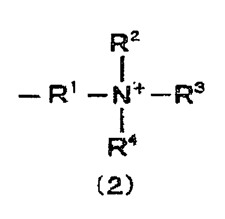 dans laquelle R¹ représente un groupe alkylène ayant de 3 à 5 atomes de carbone qui peuvent comporter un groupe hydroxyle, et chaque R², R³ et R⁴ représente un groupe alkyle ayant de 1 à 3 atomes de carbone ; et
un degré moyen de substitution avec le substituant est de 0,1 ou plus pour un motif d'acide hyaluronique.

3. Acide hyaluronique cationisé selon la revendication 1 ou 2, dans lequel l'acide hyaluronique cationisé est obtenu en faisant réagir de l'acide hyaluronique avec un agent de cationisation représenté par la formule (3) suivante : 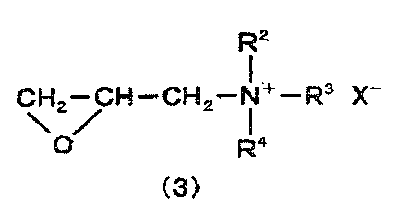 dans laquelle chacun de R², R³ et R⁴ représente un groupe alkyle ayant de 1 à 3 atomes de carbone, et X représente un atome d'halogène.

4. Complexe polyion, qui est formé avec l'acide hyaluronique cationisé selon l'une quelconque des revendications 1 à 3 et un matériau biocompatible anionique.

5. Complexe polyion selon la revendication 4, dans lequel le matériau biocompatible anionique est l'acide hyaluronique.
